(19) Europäisches Patentamt
European Patent Office
Office européen des brevets

(11) **EP 4 501 430 A1**

(12) **EUROPEAN PATENT APPLICATION**
published in accordance with Art. 153(4) EPC

(43) Date of publication:
**05.02.2025   Bulletin 2025/06**

(21) Application number: 23778898.9

(22) Date of filing: **10.02.2023**

(51) International Patent Classification (IPC):
*B01D 39/16* (2006.01)        *A01N 25/34* (2006.01)
*A01N 33/12* (2006.01)        *A01N 43/78* (2006.01)
*A01N 59/16* (2006.01)        *A01N 59/20* (2006.01)
*A01N 65/00* (2009.01)        *A01P 1/00* (2006.01)
*A61L 9/014* (2006.01)        *A61L 9/16* (2006.01)
*B03C 3/28* (2006.01)         *D06M 11/83* (2006.01)
*D06M 13/152* (2006.01)       *D06M 13/352* (2006.01)

(52) Cooperative Patent Classification (CPC):
**A01N 25/34; A01N 33/12; A01N 43/78;
A01N 59/16; A01N 59/20; A01N 65/00; A01P 1/00;
A61L 9/014; A61L 9/16; B01D 39/16; B03C 3/28;
D06M 11/83; D06M 13/152; D06M 13/352**

(86) International application number:
**PCT/JP2023/004528**

(87) International publication number:
**WO 2023/188864 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **29.03.2022   JP 2022053116**

(71) Applicant: **Toray Industries, Inc.
Tokyo 103-8666 (JP)**

(72) Inventors:
• **KATAOKA, Takuya**
  **Otsu-shi, Shiga 520-8558 (JP)**
• **HAMADA, Tsuyoshi**
  **Otsu-shi, Shiga 520-8558 (JP)**
• **NAKAMURA, Ayu**
  **Otsu-shi, Shiga 520-8558 (JP)**

(74) Representative: **Hoefer & Partner Patentanwälte
mbB
Pilgersheimer Straße 20
81543 München (DE)**

(54) **FILTRATION MEMBER FOR ANTIVIRAL AIR FILTER**

(57)     A filtration member for an antiviral air filter, having an upstream fiber layer for which the contact angle with respect to water is 60° or less, and a downstream fiber layer for which the contact angle with respect to water is 90° or greater, wherein the filtration member has an antiviral agent and a dispersant either on the upstream fiber layer or on the upstream-fiber-layer-side surface of the downstream fiber layer, and the solid fraction mass ratio of the antiviral agent and the dispersant ((mass of solid fraction of antiviral agent)/(mass of solid fraction of dispersant)) is 0.20-5.0.

The present invention provides a filtration member for an antiviral air filter in which the dust collection performance of the filter is ensured, and the amount of antiviral agent adhering to the filter is reduced and a high virus inactivation rate is achieved irrespective of changes in the environment around the filter.

EP 4 501 430 A1

**Description**

TECHNICAL FIELD

The present invention relates to a filtration member for an antiviral air filter, which is suitably used for air filter applications.

BACKGROUND ART

Conventionally, air filters have been used to remove pollen, dust, and the like in gas and purify air. For air filters for use in air purifiers and the like, filter materials made of glass fibers, paper, or nonwoven fabric are used, and the filter materials processed into pleated shapes are used for enhancing the filtration area and the filtration performance. In addition, materials with low rigidity such as a meltblown nonwoven fabric are inferior in pleat-retaining property, and thus, typically, rigid nonwoven fabrics pasted together are processed into pleated shapes for the purpose of reinforcement.

In recent years, various functions have been required to be imparted to the air filters depending on the needs of consumers, besides the removal of pollen, dust, and the like. For example, the risk of virus infection explosion is believed to be increased due to the globalization of humankind, thereby leading to an increased scale of human damage. In addition, regarding not only the expansion of the influence range due to infection but also the types of viruses, the generation of new types of viruses such as an avian influenza virus, a MERS coronavirus, an Ebola virus, and a norovirus, which lead to even the occurrence of deaths, has been observed, and solutions and countermeasures therefor have been strongly required.

However, viruses infect cells through various media, and have much smaller sizes (tens of nanometers to hundreds of nanometers) than bacteria, thus, requiring countermeasures that are different from those for bacteria that proliferate by themselves. In addition, envelope-type viruses as in influenza viruses and the like have high infectivity, and even from droplets of infected people, will infect uninfected people. As countermeasures so far, wearing masks and providing air filters have been performed against airborne infection, and protective equipment such as a glove or a gown has been used against contact infection, but the countermeasures have the problem of having insufficient functions. In particular, for preventing infection at the border, countermeasures against viral infection have been strongly desired recently for textile products surrounding humans.

In addition, there is also an increasing need for air filters that have multiple functions such as antibacterial properties, antifungal properties, and anti-allergen properties, without focusing on countermeasures against viral infection.

Patent Document 1 discloses a dust collection filter that has an extract added from tea with antimicrobial/antiviral properties. The conventional dust collection filter unit is capable of killing bacteria and fungus, but has no action of inactivating viruses that cause many diseases, and thus, there is a demand for a filter that inactivates viruses in addition to killing bacteria and fungus. In the document, however, the whole filter material is allowed to have deliquescency, thereby making bacteria and viruses to easily enter the filter from the upstream side of the air flow, but the filter material has no material that blocks the flow of bacteria and viruses, and thus causes bacteria and viruses to come out to the downstream side, thereby causing the viruses to spread again.

Patent Document 2 discloses a harmful substance removing material carrying an antibody that removes harmful substances under a gas phase atmosphere, with the atmosphere around the antibody being adjusted to a humidity at which the antibody exhibits activity. In the document, the antibody is used for a method for inactivating viruses, but the antibody is easily denatured by a change in environment, and is not suitable for air filters for applications such as an automobile cabin filter, which have the possibility of reaching a high temperature and a high humidity. In addition, the virus inactivation rate varies depending on the humidity, and the antibody is thus less effective if the material is used at the time when infection with influenza viruses and the like is increased with low humidity.

PRIOR ART DOCUMENTS

PATENT DOCUMENTS

  Patent Document 1: Japanese Patent Laid-open Publication No. 10-315
  Patent Document 2: Japanese Patent Laid-open Publication No. 2004-313755

SUMMARY OF THE INVENTION

PROBLEMS TO BE SOLVED BY THE INVENTION

Accordingly, the present invention provides a filtration member for an antiviral air filter, which ensures the dust collection performance of the filter, and irrespective of changes in the environment around the filter, achieves a high virus inactivation rate even when the adhesion amount of antiviral agent is reduced.

SOLUTIONS TO THE PROBLEMS

A filtration member for an antiviral air filter, including an upstream fiber layer for which the contact angle with respect to water is 60° or less, and a downstream fiber layer for which the contact angle with respect to water is 90° or greater, where the filtration member has an antiviral agent and a dispersant either on the upstream fiber layer or on the upstream-fiber-layer-side surface of the downstream fiber layer, and the solid fraction mass ratio of the antiviral agent to the dispersant ((solid fraction mass of antiviral agent)/(solid fraction mass of dispersant)) is 0.20 to 5.0.

The filtration member for an antiviral air filter according to the present invention preferably satisfies any one of the following (1) to (11):

(1) The basis weight of the solid fraction mass of the antiviral agent is 0.01 to 4.0% with respect to the basis weight of the upstream fiber layer or the downstream fiber layer including the antiviral agent.
(2) The downstream fiber layer is an electret nonwoven fabric.
(3) The antiviral agent includes a compound including a metal atom.
(4) The antiviral agent includes an organic compound with ionicity.
(5) The antiviral agent includes a naturally derived component or a derivative thereof.
(6) The antiviral agent further has an antibacterial property, an antifungal property, or an anti-allergen property.
(7) The dispersant is a nonionic surfactant.
(8) Besides the antiviral agent, a functional agent with an antibacterial property, an antifungal property, or an anti-allergen property is further included on the upstream fiber layer or on the upstream-fiber-layer-side surface of the downstream fiber layer.
(9) An intermediate layer including an adsorbent is included between the upstream fiber layer and the downstream fiber layer.
(10) The filtration member is obtained by applying a processing liquid including an antiviral agent and a dispersant to the upstream fiber layer or the upstream-fiber-layer-side surface of the downstream fiber layer by a processing method of impregnation or spraying.
(11) The processing liquid has pH of 3.0 or more and 6.0 or less.

In addition, the filtration member for an antiviral air filter according to the present invention is fabricated by a fabrication method of: applying a processing liquid with pH of 3.0 or more and 6.0 or less, including the antiviral agent and the dispersant, to the upstream fiber layer or the upstream-fiber-layer-side surface of the downstream fiber layer by a processing method of impregnation or spraying; and stacking the upstream fiber layer or the downstream fiber layer on each other, thereby providing the upstream fiber layer for which the contact angle with respect to water is 60° or less and the downstream fiber layer for which the contact angle with respect to water is 90° or more.

EFFECTS OF THE INVENTION

According to the present invention, the air filter has a configuration where the upstream fiber layer is made hydrophilic, whereas the downstream fiber layer is made water-repellent, such that moisture in the air containing a virus is made more likely to be taken into the filtration member without remaining on the surface of the upstream fiber layer, and remains on the surface of the downstream fiber layer to confine the virus and then increase the rate of contact with the antiviral agent that inactivates the virus, and the interaction with the antiviral agent is alleviated with the dispersant to attach the antiviral agent uniformly to the fibers without decreasing the antiviral performance due to aggregation, and thus further increase the virus inactivation efficiency, thereby allowing the antiviral property to be sufficiently exhibited even with a small adhesion amount.

EMBODIMENTS OF THE INVENTION

Hereinafter, the present invention will be described in detail.
A filtration member for an antiviral air filter according to the present invention included an upstream fiber layer for which the contact angle with respect to water is 60° or less, and a downstream fiber layer for which the contact angle with respect to water is 90° or greater, the filtration member has an antiviral agent and a dispersant either on the upstream fiber layer or on the upstream-fiber-layer-side surface of the downstream fiber layer, and the solid fraction mass ratio of the antiviral agent to the dispersant ((solid fraction mass of antiviral agent)/(solid fraction mass of dispersant)) is 0.20 to 5.0.

The filtration member for an air filter according to the present invention, which employs such a configuration, is considered to exhibit the effect of the present invention in accordance with the following mechanism. First, as mentioned above, the air filter has a configuration where the upstream fiber layer is made hydrophilic, whereas the downstream fiber layer is made water-repellent for causing moisture in the air containing a virus to be confined in the filtration member, such that moisture in the air containing the virus is made more likely to permeate and taken into the filtration member without remaining on the surface of the upstream fiber layer, whereas the virus is made less likely to permeate the downstream fiber layer to cause the virus to remain on the upstream-layer-side surface of the downstream fiber layer. Accordingly, the antiviral agent is preferably attached to the upstream fiber layer or the upstream-fiber-layer-side surface of the downstream fiber layer, and the antiviral agent may be attached to both the upstream fiber layer and the upstream-fiber-layer-side surface of the downstream fiber layer.

The hydrophilicity and water repellency of the fiber layers are determined based on a contact angle with respect to water. The contact angle refers to an angle made by a liquid surface and a solid surface at a site with a free surface of a stationary liquid in contact with a solid wall (taking an angle inside the liquid). The fiber layer is determined to be hydrophilic when the contact angle with respect to water with the fiber layer surface kept horizontal is 60° or less, whereas the fiber layer is determined to be water-repellent when the contact angle is 90° or more. Further, water may permeate the fiber layer to result in no water droplet on the surface of the fiber layer, and this case is also determined to be hydrophilic. Moisture in the air containing viruses is more likely to be compatible with the hydrophilic fiber layer, and in particular, envelope-type viruses typified by influenza viruses with hydrophilic surfaces are more likely to be compatible with the hydrophilic fiber layer. In contrast, viruses are made less likely to penetrate the water-repellent fiber layer, and are less likely to be released outside the filter.

The antiviral agent has a high biocidal property and high reactivity, and thus, the aggregation by interaction with the antiviral agent has a possibility of significantly decreasing the high antiviral performance. The interaction with the antiviral agent is alleviated with the dispersant to disperse the agent, thereby allowing the antiviral agent to be uniformly attached to the filtration member without decreasing the original antiviral performance. These effects can maximize the contact efficiency between the virus and the antiviral agent.

In addition, the basis weight of the solid fraction mass of the antiviral agent is preferably 0.01 to 4.0%, more preferably 0.05 to 2.0% with respect to the basis weight of the upstream fiber layer or downstream fiber layer including the antiviral agent. The basis weight of 0.01% or more allows high antiviral performance to be achieved, and the basis weight of 4.0% or less allows the aggregation of the antiviral agent to be further suppressed.

Examples of the material of fibers constituting the upstream fiber layer and the downstream fiber layer include glass, polyolefin, polyester, polyamide, vinylon, and cellulose pulp, examples of the form of the fiber layer include paper, nonwoven fabrics, knitted fabrics, and network products, and among these examples, air-laid nonwoven fabrics, wet nonwoven fabrics, spun-bonded nonwoven fabrics, meltblown nonwoven fabrics, thermally bonded nonwoven fabrics, stitch bonded nonwoven fabrics, needle-punched nonwoven fabrics, spunlace nonwoven fabrics, and the like are preferred from the viewpoint of handleability. In particular, the downstream fiber layer that plays a role of keeping any virus from release to the outside as much as possible is preferably a meltblown non-woven fabric, and from the viewpoint of having high dust removal performance, the meltblown non-woven fabric is preferably an electret nonwoven fabric subjected to electret processing. In addition, the meltblown non-woven fabric preferably has an average fiber diameter of 6 $\mu$m or less, more preferably 3 $\mu$m or less. These make it possible to further prevent the virus from flowing out to the downstream fiber layer and the subsequent layers.

For the antiviral agent, known antiviral agents can be used, and examples thereof include metal nanoparticles such as silver and copper, metal oxides such as a copper oxide and a copper sulfate, supports obtained by adding metals such as silver and copper to carriers such as zeolite, a silica gel, glass, a calcium phosphate, a zirconium phosphate, a calcium silicate, a magnesium aluminometasilicate, a potassium titanate, and a zinc oxide, alcohol-based compounds, phenol-based compounds, quaternary ammonium salts, benzoic acids, chlorhexidine, sorbic acids, carboxylic acids, aldehydes, ionic surfactants, components or derivatives derived from natural products, such as plant essential oils, catechins, and tannins.

In particular, an antiviral agent including metal atoms is compatible with fibers and is easily attached to and blended into the fibers, and thus preferred as the antiviral agent according to the present invention. In addition, the antiviral agent including metal atoms has slowly acting efficacy, and thus fails to cause the inactivation of a virus to proceed at a low frequency of contact with the virus, but in contrast, the configuration of confining the virus in the filtration member according to the present invention increases the frequency of contact between the virus and the antiviral agent, thereby making the efficacy acting more.

In addition, an antiviral agent that is an organic compound with ionicity is also preferred for the present invention, and the organic compound with ionicity has high reactivity with a virus, thus has a high inactivation rate at the time of contact with the virus, and allows efficient contact with the virus in the present invention in which the antiviral agent is uniformly dispersed in the filtration member.

In addition, the air filter is highly frequently replaced and cleaned, and an antiviral agent containing a natural product such as catechins or tannins with high skin irritation and excellent safety or a derivative thereof is also preferred for the present invention.

The antiviral agent for use in the present invention preferably further has an antibacterial property, an antifungal property, or an anti-allergen property. This makes it possible to suppress deterioration over time and maintain the performance as an air filter for a longer time.

In addition, when the antiviral agent further has an antibacterial property, an antifungal property, or an anti-allergen property, there is no need to add another different substance for exhibiting these properties. Thus, there is no possibility of functional degradation due to the interaction between the antiviral agent and the different substance, which is preferred.

Further, examples of the antiviral agent with an antibacterial property include compounds including metal atoms, such as silver-copper-zinc-supported zeolite. Furthermore, examples of the antiviral agent with an antifungal property include an organic compound with ionicity, such as a quaternary ammonium salt. In addition, examples of the antiviral agent with an anti-allergen property include naturally derived components such as natural plant extracts or derivatives thereof.

In the present invention, besides the antiviral agent, a functional agent with an antibacterial property, an antifungal property, or an anti-allergen property may be further included on the upstream fiber layer or on the upstream-fiber-layer-side surface of the downstream fiber layer. This protects the filtration member from adhering bacteria, fungus, and allergen, thereby making it possible to suppress deterioration over time and maintain the performance as an air filter for a longer time.

For the antibacterial agent, known antibacterial agents can be used, and examples thereof include metal nanoparticles such as zinc, zeolite, a silica gel, glass, a calcium phosphate, a zirconium phosphate, a calcium silicate, a magnesium aluminometasilicate, a potassium titanate, supports obtained by adding metals such as zinc to carriers such as a zinc oxide, inorganic antibacterial agents, for example, metal oxides such as a zinc oxide and a titanium dioxide, carbamate-based agents such as 3-iodo-2-propynyl butyl carbamate, and a benzalkonium chloride.

For the antifungal agent, known antifungal agents can be used, and examples thereof include benzimidazole-based agents such as 2-(4-thiazolyl)-benzimidazole (thiabendazole) and methyl-2-benzimidazole carbamate (carbendazim), sulfamide-based agents such as N-dichlorofluoromethylthio-N',N'-dimethyl-N-phenylsulfamide (dichlofluanid) and N-dichlorofluoromethylthio-N',N'-dimethyl-N-p-tolylsulfamide (trifluanid), 2-bromo-2 nitropropane-1,3-diol, and orthophenylphenol. In addition, some of the antibacterial agents described above have an antifungal property, and can also be used as appropriate.

For the anti-allergen agent, known anti-allergen agents can be used, and examples thereof include alkaline earth metal salts such as calcium salts and strontium salts, zirconium salts such as zirconyl chloride, a zirconium hydroxide, and a zirconium carbonate, and aluminum salts such as alum and an aluminum sulfate. In addition, some of the antibacterial agents and antifungal agents described above have an anti-allergen property, and can also be used as appropriate.

In addition, the attachment amount is preferably 0.05 to 2.0%, more preferably 0.1 to 1.0% with respect to the basis weight of the upstream fiber layer or downstream fiber layer including the antiviral agent. The attachment amount is 0.05% or more, thereby allowing the effect as a functional agent to be sufficiently exhibited, and the attachment amount is 2.0% or less, thereby allowing interference with the highly reactive antiviral agent to be reduced to keep the antiviral property from being decreased. In addition, the high water solubility of the functional agent causes the functional agent to be eluted with moisture attached to the filtration member, and thus, it is preferable to use a water-insoluble functional agent.

The filtration member for an antiviral air filter according to the present invention has a dispersant on the upstream fiber layer or on the upstream-fiber-layer-side surface of the downstream fiber layer. This makes it possible to more uniformly disperse the antiviral agent. In addition, the solid fraction mass ratio between the antiviral agent and the dispersant (solid fraction mass of antiviral agent/solid fraction mass of dispersant) is 0.20 to 5.0, preferably 0.25 to 2.5. In the case of a ratio lower than 0.20, the antiviral agent is aggregated without being well dispersed, and in the case of a ratio higher than 5.0, the excessive dispersant causes the antiviral property of the antiviral agent to be decreased.

Common examples of the dispersant include polycarboxylic acid-based dispersants, naphthalene sulfonic acid-formalin condensation-based dispersants, polyethylene glycols, polycarboxylic acid partial alkyl ester-based dispersants, polyether-based dispersants, polyalkylene polyamine-based dispersants, alkyl sulfonic acid-based dispersants, quaternary ammonium-based dispersants, higher alcohol-alkylene oxide-based dispersants, polyhydric alcohol ester-based dispersants, polyalkyl polyamine-based dispersants, polyphosphate-based dispersants, carboxymethyl celluloses, and polysaccharide.

In particular, the dispersant is preferably a nonionic surfactant, and furthermore, preferably a low molecular weight compound. It is to be noted that the low molecular weight compound is defined as a compound that has a molecular weight of 1000 or less. The dispersant is nonionic, thereby allowing the influence on the highly reactive antiviral agent to be further reduced. In addition, the dispersant is a low molecular weight compound, thereby making the contact between the antiviral agent and the virus less likely to be hindered.

In the case of using as an air filter, also conceivable is an embodiment including an intermediate layer containing an adsorbent between the upstream fiber layer and the downstream fiber layer. The presence of the intermediate layer containing an adsorbent makes it possible to encapsulate an odorous organic low molecular weight compound such as ammonia or toluene without releasing the compound from the filtration member, thereby allowing an improvement in air cleaning performance. In addition, because of the configuration in which the virus is likely to remain in the intermediate layer, the virus can be confined more inside. Common examples of the adsorbent include activated alumina, activated carbon, activated carbon fibers, silica gel, and zeolite, and the adsorbent is more preferably activated carbon from the viewpoint of an odor substance adsorption effect. In addition, preferred is a configuration in which the antiviral agent can be attached to the inside or surface of the adsorbent, and the adsorbent with the antiviral agent thereto is introduced into the intermediate layer. The virus remaining in the intermediate layer can be efficiently inactivated. In addition, it is also possible to attach not only the antiviral agent, but also a functional agent with an antibacterial property, an antifungal property, or an anti-allergen property. The inside of the intermediate layer can be prevented from acting as a source for propagation of bacteria or fungus. Alternatively, the adsorbent may be attached to the upstream fiber layer or the downstream fiber layer, rather than the intermediate layer.

It is also possible to introduce a flame retardant aid into the intermediate layer, and common examples thereof include phosphorus-based flame retardants, nitrogen-based flame retardants, halogen-based flame retardants, bromine-based flame retardants, and composites thereof, a magnesium hydroxide, an aluminum hydroxide, antimony-based flame retardants, thermally expandable graphite, and silicone-based flame retardants. The flame retardant aid can be also attached to the fibers of the upstream fiber layer or downstream fiber layer, but possibly reacts with the antiviral agent, and is preferably attached to the layer without the antiviral agent attached thereto. Alternatively, the flame retardant may be attached to the upstream fiber layer or the downstream fiber layer, rather than the intermediate layer, or flame-retardant fibers with the flame retardant blended into the fibers may be used. In particular, when the flame retardant has high water solubility, the flame retardant is eluted with moisture attached to the filtration member, and thus, it is preferable to use a water-insoluble flame retardant. In addition, a phosphoric acid flame retardant without any halogen element used in consideration of environmental effects can be used regardless of the application of the filtration member, is often a compound that is stable at pH around 3.0 to 6.0, and has good compatibility with many antiviral agents. In particular, it is more preferable to use a water-insoluble phosphorus-based flame retardant.

In the case of a filtration member with the upstream fiber layer and the downstream fiber layer bonded to each other, the peel strength of the two layers is preferably 0.5 N or more as measured in accordance with JIS L 1086 (2013) standard 7.10.1. The peel strength is 0.5 N or more, thereby making delamination unlikely to occur between the upstream fiber layer and the downstream fiber layer, and reducing the influence at the time of molding or high-order processing. It is preferable to use an adhesive for bonding the upstream fiber layer and the downstream fiber layer, and common examples of the adhesive include aqueous adhesives such as a melamine resin, a phenol resin, and a vinyl acetate resin, chemical reaction-based adhesives such as an epoxy resin and a urethane resin, solvent-based adhesives, and a hot melt-based adhesives. In particular, a hot melt-based adhesive is preferable in terms of the process passability, workability, and cost of the filtration member.

In the case of bonding only the upstream fiber layer and the downstream fiber layer, the adhesive is preferably 1.0 to 20.0 g/m$^2$. When the adhesive is 1.0 g/m$^2$ or more, the upstream fiber layer and the downstream fiber layer are more sufficiently bonded, and when the adhesive is 20.0 g/m$^2$ or less, the antiviral agent attached to the upstream fiber layer or the upstream-fiber-layer-side surface of the downstream fiber layer is covered with the adhesive, thereby allowing the antiviral performance to be further kept from being decreased.

In addition, when at least one intermediate layer is included between the upstream fiber layer and the downstream fiber layer, the adhesive preferably is 5% to 40% in basis weight with respect to the basis weight of the intermediate layer. When the adhesive is 5% or more, the upstream fiber layer and the downstream fiber layer are more sufficiently bonded, and when the adhesive is 40% or less, the antiviral agent attached to the upstream fiber layer or the upstream-fiber-layer-side surface of the downstream fiber layer is covered with the adhesive, thereby allowing the antiviral performance to be further kept from being decreased. In particular, when the intermediate layer includes therein no fibers, the adhesive is more preferably 15% to 40%.

As for the method for attaching the antiviral agent and the dispersant to the upstream fiber layer or the downstream fiber layer according to the present invention, preferably, the antiviral agent and the dispersant are mixed in an aqueous solvent in the same bath to form a processing liquid, and the fibers are impregnated with the processing liquid, or the processing liquid is applied by spraying to the fibers. In addition, the antiviral agent and the dispersant may be introduced at the time of producing the fiber layer by blending the antiviral agent and the dispersant into molten fibers or mixing the antiviral agent and the dispersant with a binder resin. In the case of using another functional agent, the antiviral agent and the dispersant may be mixed with an aqueous solvent to form a processing liquid, the fibers may be impregnated with the processing liquid, or the processing liquid may be applied by spraying to the fibers, and then, the fibers may be impregnated with a processing liquid formed by mixing the functional agent with an aqueous solvent, or the processing liquid may be applied by spraying to the fibers. In addition, the antiviral agent, the dispersant, and the functional agent may be mixed in an aqueous solvent in the same bath to form a processing liquid, and the fibers may be impregnated with the processing liquid, or the processing liquid may be applied by spraying to the fibers. In addition, as with the antiviral agent, the functional agent may be introduced at the time of producing the fiber layer by blending the functional agent into molten fibers or mixing the functional agent with a binder resin. In the case of mixing the functional agent with the antiviral agent in the same bath, there is concern such as agent aggregation if the agents have poor compatibility with each other, and thus, the functional agent is preferably attached in a separate step. In impregnating the fibers with the processing liquid or applying the processing liquid by spraying to the fibers, it is preferable to stir the processing liquid until immediately before the impregnation or the application, thereby allowing the antiviral agent to be kept from being aggregated. For improving the stability of the processing liquid, the pH of the processing liquid is preferably 3.0 or more and 6.0 or less, more preferably 3.5 or more and 5.0 or less. The pH of 3.0 or more has a lower possibility of damaging the fibers because of the appropriate acidity of the processing liquid, and the pH of 6.0 or less has a lower possibility of decreasing the solution stability of each agent of the processing liquid over time.

Typical processing methods include a fabrication method of applying a processing liquid with pH of 3.0 or more and 6.0 or less, including the antiviral agent and the dispersant, to the upstream fiber layer or the upstream-fiber-layer-side surface of the downstream fiber layer by a processing method of impregnation or spraying, and stacking the upstream fiber layer or the downstream fiber layer on each other, thereby providing the upstream fiber layer for which the contact angle with respect to water is 60° or less and the downstream fiber layer for which the contact angle with respect to water is 90° or more.

Examples of the aqueous solvent include water alone, or an aqueous solution containing a water-soluble organic solvent such as lower alcohols such as methanol and ethanol, lower ketones such as acetone and methyl ethyl ketone, lower carboxylic acids such as an acetic acid, and glycols such as an ethylene glycol, a propylene glycol, and a diethylene glycol, and the aqueous solvent can be selected depending on the types of antiviral agent and the like.

The method for drying the filtration member with the processing liquid attached thereto is not particularly limited, but a hot air drying method, a Yankee drum method, or the like is preferably employed. The drying temperature is preferably 100 to 230°C, more preferably 110 to 150°C. The drying temperature is 100°C or higher, thereby allowing the drying time to be sufficiently shortened. In addition, the drying temperature is 230°C or lower, thereby allowing the antiviral agent, the dispersant, and the functional agent to be further kept from being decomposed, and allowing the antiviral property to be sufficiently exhibited.

The filtration member according to the present invention is suitably used for air filter applications, particularly as filters for air purifiers and cabin filters for automobiles, and thus, the filtration member can be processed into shapes such as a pleated shape and then suitably used as filter units. For achieving the retention of the pleated shape of the filter, it is more preferable to use a fiber layer that has, as the rigidity of the filtration member, a bending resistance of 300 mg or more as measured by a Gurley method.

EXAMPLES

Hereinafter, the present invention will be described more specifically with reference to examples, but the present invention is not necessarily limited to these examples. In addition, the results and the like of the respective examples and comparative examples are shown in Tables 1 and 2. First, related measurement methods and evaluation methods will be described.

(1) Basis weight

The mass of a sample was determined by allowing the sample to stand at room temperature of 24°C and 60%RH for 8 hours or longer, and converted to the mass per $1m^2$ from the area of the sample to determine the basis weight of the sample. The minimum area of the sample was adjusted to be $0.01m^2$ or more.

(2) Adhesion amount

As for the amount of the antiviral agent, dispersant, and functional agent adhering to the fiber layer, the total adhesion amount was calculated by measuring, under the condition (1), the basis weight of the fiber layer before attaching or blending the antiviral agent, the dispersant, and the functional agent, and the basis weight of the fiber layer after attaching or blending the antiviral agent, the dispersant, and the functional agent, and determining the difference therebetween. Next, the adhesion amounts of all of the components were each calculated by multiplying the total adhesion amount by the solid fraction mass ratios of the both substances.

(3) Hydrophilicity confirmation test

Onto the surface of the fiber layer of 100 mm × 100 mm, 50 μL of distilled water was added dropwise from a height of 5 mm, and the permeability was visually confirmed. The state of water droplets on the fiber layer 5 seconds after the dropwise addition was evaluated in accordance with the following criteria.

⊙: The distilled water permeating the fiber layer, without any water droplet on the fiber layer.
∘: Water droplets on the fiber layer, with the contact angle of 60° or less.
△: Water droplets on the fiber layer, with the contact angle larger than 60° and smaller than 90°.
×: Water droplets on the fiber layer, with the contact angle of 90° or more.

(4) Antiviral test

In accordance with JIS L 1922 (2016) (Determination of antiviral activity of textile products (plaque measurement method)), 0.4 g of the sample was put in a vial, and 0.2 ml of a virus liquid was inoculated into the sample, the vial bottle was allowed to stand at 25°C for 2 hours, then, 20 ml of an SCDLP medium was added to wash out the virus from the sample, and the viral infectivity titer of the liquid washed out was measured by a plaque measurement method. The antiviral activity was calculated in accordance with the following formula. In addition, regarding the blank, the same operation as mentioned above was performed without using the sample, and the viral infectivity titer was calculated.

Antiviral activity = log (infectivity titer after 2 --> hour action of blank) - log (infectivity titer after 2 hour action of sample)

For the blank, a standard cloth (cotton) is typically used. The type of the virus used in the test is influenza virus (H1N1).
The calculated antiviral activity was evaluated in accordance with the following criteria.

⊙: The antiviral activity of 3.0 or more
∘: The antiviral activity of 2.0 or more and less than 3.0
△: The antiviral activity of 1.0 or more and less than 2.0
×: The antiviral activity of less than 1.0

(5) Antibacterial test

In accordance with JIS L 1902 (2015) (Determination of antibacterial activity and efficacy of textile products (bacteria liquid absorption method)), 0.4 g of the sample was put into a vial, 0.2 ml of a test bacteria liquid was added dropwise thereto, the vial was then subjected to culture at 37°C for 18 to 24 hours, 20 ml of a washing liquid was then added to wash out the test bacteria from the test piece, and the viable bacteria count in the washing liquid was measured by a pour plate culture method. The antibacterial activity was calculated in accordance with the following formula.

Antibacterial activity = [log (viable bacteria count after culture of blank) - log (viable bacteria count after inoculation into blank)] - [log (viable bacteria count after culture of sample) - log (viable bacteria count after inoculation into sample)]

In addition, regarding the blank, the same operation as mentioned above was performed without using the sample, and the viable bacteria count was calculated. For the blank, a standard cloth (cotton) was typically used. The type of the bacteria used in the test is Staphylococcus aureus.
The calculated antibacterial activity was evaluated in accordance with the following criteria.

⊙: The antibacterial activity of 3.0 or more
∘: The antibacterial activity of 2.0 or more and less than 3.0
△: The antibacterial activity of 1.0 or more and less than 2.0
×: The antibacterial activity of less than 1.0

(6) Antifungal test

In accordance with JIS Z2911 (2018) (Methods of test for fungus resistance (textile product test, wet method)), a mixed spore suspension of fungus was inoculated into the sample, and after culture for a certain period of time, the state of growth of hyphae produced on the surface of the sample was observed with the naked eye or a microscope. The fungus used in the test was a mixture of four species of Aspergillus niger, Penicillium citrinum, Chaetomium globosum, and Myrothecium verrucaria in equal amounts, and the size of the sample was about 5 cm × 5 cm.

The observed results were evaluated in accordance with the following criteria.

⊙: No growth of hyphae observed in the inoculated part of the sample, with a recognizable halo in the medium with the sample placed thereon

∘: No growth of hyphae observed in the inoculated part of the sample, with no halo recognized in the medium with the sample placed thereon

△: The part of hypha growth recognized in the inoculated part of the sample is 1/3 or less of the total area

✕: The part of hypha growth recognized in the inoculated part of the sample is more than 1/3 of the total area

(7) Anti-allergen test (allergen reduction rate test)

Each of target allergens (cedar pollen allergen Cryj 1, mite allergen Derf 2) was dissolved in a phosphate buffer so as to be 100 ng/ml, thereby preparing respective allergen solutions.

Next, the fiber layer was cut into 70 mg each, the cut fiber layers were put into microtubes, 1 ml of the allergen solution was added to each of the microtubes, the microtubes were shaken at 4°C for 5 hours, and allowed to stand for 16 hours to develop reactions. The allergen concentration of the allergen solution reacted with the sample was measured by a sandwich ELISA method. The sandwich ELISA method was performed in accordance with the following procedure.

An antibody against the allergen (antigen) was immobilized on each well of a microplate. Thereafter, the antibody immobilized was washed three times with post-coating (1% BSAPBS) and PBS (containing 0.05% Tween 20) (the same washing method applies hereinafter), and the sample and the standard allergen were added thereto. After washing, a labeled antibody against the allergen was added, and after washing, streptavidin HRPO was added thereto. After washing, o-phenylenediamine was added, $H_2SO_4$ was added to stop the reaction, and then, the absorbance at 490 nm was measured with a microplate reader.

Separately, a calibration curve of absorbance-allergen concentration was created with a standard allergen solution, the allergen concentration was calculated, and the allergen reduction rate was calculated by the following formula.

$$\text{Allergen reduction rate (\%)} = (B - A)/B \times 100$$

A: the allergen concentration in the allergen solution after the sample reaction
B: the allergen concentration in the allergen solution of the sample unreacted

The observed results were evaluated in accordance with the following criteria.

⊙: The allergen reduction rate of 98% or more
∘: The allergen reduction rate of 90% or more and less than 98%.
△: The allergen reduction rate of 50% or more and less than 90%.
✕: The allergen reduction rate of less than 50%.

[Example 1]

(Upstream fiber layer)

30 parts by mass of polyester short fibers of 16 dt in fineness (polyethylene terephthalate, melting point: 265°C), 20 parts by mass of thermally fused polyester short fibers of 8 dt in fineness (core part: polyethylene terephthalate, melting point: 265°C, sheath part: modified polyester, melting point: 155°C), and 50 parts by mass of thermally fused polyester short fibers of 4.4 dt in fineness (core part: polyethylene terephthalate, melting point: 265°C, sheath part: modified polyester, melting point: 180°C) were mixed, and passed through a cotton opening machine and a carding machine to spin a web, and the web was superposed by cross wrap to reach a target mass, and passed through an air-through heating furnace with a conveyor to obtain a thermally bonded nonwoven fabric of 70 g/m² in basis weight and 0.4 mm in thickness.

A processing liquid at pH 7.0 obtained by mixing silver-copper-zinc-supported zeolite ("Agion" (registered trademark) AM Slurry manufactured by Sciessent LLC) as an antiviral agent and a nonionic surfactant ("TEXPORT SN-10" manufactured by NICCA CHEMICAL CO., LTD.) as a dispersant in water was put in an impregnation tank, and the thermally bonded nonwoven fabric was passed through the impregnation tank to be impregnated with the processing liquid, and then passed through an air-through heating furnace to fabricate an upstream fiber layer. The concentration of the processing liquid was adjusted such that the adhesion amount of the silver-copper-zinc-supported zeolite was 3.5 g/m$^2$ and such that the adhesion amount of the nonionic surfactant was 3.5 g/m$^2$.

Accordingly, the solid fraction mass ratio between the antiviral agent and the dispersant (solid fraction mass of antiviral agent/the solid fraction mass of dispersant) was 3.5/3.5 = 1.0, and the basis weight of the solid fraction mass of the antiviral agent was 3.5/70 = 0.05 (5.0%) with respect to the basis weight of the upstream fiber layer.

(Downstream fiber layer)

With the use of a polypropylene resin, a downstream fiber layer of 30.0 μm in average fiber diameter and 30 g/m$^2$ in basis weight was obtained by a spunbonding method.

(Filtration member)

To the upstream fiber layer and downstream fiber layer obtained, a hot-melt resin made of a polyethylene was dispersed so as to be 6.0 g/m$^2$, and the fiber layers were combined with each other to obtain a filtration member for an air filter.

[Example 2]

(Upstream fiber layer)

An upstream fiber layer was obtained in the same manner as in Example 1 except that the concentration of the processing liquid was adjusted such that the adhesion amount of the silver-copper-zinc-supported zeolite was 0.7 g/m$^2$ and such that the nonionic surfactant was 0.7 g/m$^2$.

Accordingly, the solid fraction mass ratio between the antiviral agent and the dispersant (solid fraction mass of antiviral agent/the solid fraction mass of dispersant) was 0.7/0.7 = 1.0, and the basis weight of the solid fraction mass of the antiviral agent was 0.7/70 = 0.01 (1.0%) with respect to the basis weight of the upstream fiber layer.

(Downstream fiber layer)

With the use of a polypropylene resin, a nonwoven fabric of 2.0 μm in average fiber diameter and 30 g/m$^2$ in basis weight was obtained by a melt-blowing method. Then, while running the obtained nonwoven fabric along the surface of water in a water tank that was supplied with pure water, a slit-shaped suction nozzle was brought into contact with the surface of the nonwoven fabric to suck the water, thereby causing the water to permeate the whole fiber layer, and after draining the water, the fiber layer was naturally dried, thereby providing an electretized downstream fiber layer.

(Filtration member)

The fiber layers were combined in the same manner as in Example 1 to obtain a filtration member for an air filter.

[Example 3]

(Upstream fiber layer)

A thermally bonded nonwoven fabric was obtained in the same manner as in Example 1, a processing liquid at pH 7.0 obtained by mixing a quaternary ammonium salt ("Niccanon RB-40" manufactured by NICCA CHEMICAL CO., LTD.) as an antiviral agent and a nonionic surfactant ("TEXPORT SN-10" manufactured by NICCA CHEMICAL CO., LTD.) as a dispersant in water was put in an impregnation tank, and the thermally bonded nonwoven fabric was passed through the impregnation tank to be impregnated with the processing liquid, and then passed through an air-through heating furnace to fabricate an upstream fiber layer. The concentration of the processing liquid was adjusted such that the adhesion amount of the quaternary ammonium salt was 0.7 g/m$^2$ and such that the adhesion amount of the nonionic surfactant was 0.7 g/m$^2$.

Accordingly, the solid fraction mass ratio between the antiviral agent and the dispersant (solid fraction mass of antiviral agent/the solid fraction mass of dispersant) was 0.7/0.7 = 1.0, and the basis weight of the solid fraction mass of the antiviral agent was 0.7/70 = 0.01 (1.0%) with respect to the basis weight of the upstream fiber layer.

(Downstream fiber layer)

A downstream fiber layer was obtained in the same manner as in Example 2.

(Filtration member)

The fiber layers were combined in the same manner as in Example 1 to obtain a filtration member for an air filter.

[Example 4]

(Upstream fiber layer)

A thermally bonded nonwoven fabric was obtained in the same manner as in Example 1, a processing liquid at pH 7.0 obtained by mixing a natural plant extract ("Allersave T-70" manufactured by SC Environmental Science Co., Ltd.) as an antiviral agent and a nonionic surfactant ("TEXPORT SN-10" manufactured by NICCA CHEMICAL CO., LTD.) as a dispersant in water was put in an impregnation tank, and the thermally bonded nonwoven fabric was passed through the impregnation tank to be impregnated with the processing liquid, and then passed through an air-through heating furnace to fabricate an upstream fiber layer. The concentration of the processing liquid was adjusted such that the adhesion amount of the natural plant extract was 0.7 $g/m^2$ and such that the adhesion amount of the nonionic surfactant was 0.7 $g/m^2$.

Accordingly, the solid fraction mass ratio between the antiviral agent and the dispersant (solid fraction mass of antiviral agent/the solid fraction mass of dispersant) was 0.7/0.7 = 1.0, and the basis weight of the solid fraction mass of the antiviral agent was 0.7/70 = 0.01 (1.0%) with respect to the basis weight of the upstream fiber layer.

(Downstream fiber layer)

A downstream fiber layer was obtained in the same manner as in Example **2.**

(Filtration member)

The fiber layers were combined in the same manner as in Example 1 to obtain a filtration member for an air filter.

[Example 5]

(Upstream fiber layer)

A thermally bonded nonwoven fabric was obtained in the same manner as in Example 1, a processing liquid at pH 7.0 obtained by mixing silver-copper-zinc-supported zeolite ("Agion" AM Slurry manufactured by Sciessent LLC) as an antiviral agent, a nonionic surfactant ("TEXPORT SN-10" manufactured by NICCA CHEMICAL CO., LTD.) as a dispersant, and a thiabendazole ("Synthol M-30" manufactured by SC Environmental Science Co., Ltd.) to serve as an antifungal agent as a functional agent other than the antiviral agent in water was put in an impregnation tank, and the thermally bonded nonwoven fabric was passed through the impregnation tank to be impregnated with the processing liquid, and then passed through an air-through heating furnace to fabricate an upstream fiber layer. The concentration of the processing liquid was adjusted such that the adhesion amount of the silver-copper-zinc-supported zeolite was 0.7 $g/m^2$, such that the adhesion amount of the nonionic surfactant was 0.7 $g/m^2$, and such that the adhesion amount of the thiabendazole was 0.7 $g/m^2$.

Accordingly, the solid fraction mass ratio between the antiviral agent and the dispersant (solid fraction mass of antiviral agent/the solid fraction mass of dispersant) was 0.7/0.7 = 1.0, and the basis weight of the solid fraction mass of the antiviral agent was 0.7/70 = 0.01 (1.0%) with respect to the basis weight of the upstream fiber layer, and the basis weight of the solid fraction mass of the antifungal agent was 0.7/70 = 0.01 (1.0%) with respect to the basis weight of the upstream fiber layer.

(Downstream fiber layer)

A downstream fiber layer was obtained in the same manner as in Example 2.

(Filtration member)

The fiber layers were combined in the same manner as in Example 1 to obtain a filtration member for an air filter.

[Example 6]

(Upstream fiber layer)

An upstream fiber layer was obtained in the same manner as in Example 2.

(Downstream fiber layer)

An electretized nonwoven fabric was obtained in the same manner as in Example 2, and a processing liquid at pH 7.0 obtained by mixing a thiabendazole ("Synthol M-30" manufactured by SC Environmental Science Co., Ltd.) to serve as an antifungal agent as a functional agent other than the antiviral agent in water was applied by spraying, and dried at 110°C to obtain a downstream fiber layer. The concentration of the processing liquid was adjusted such that the adhesion amount of the thiabendazole was 0.3 g/m$^2$. Accordingly, the basis weight of the solid fraction mass of the antifungal agent was 0.3/30 = 0.01 (1.0%) with respect to the basis weight of the downstream fiber layer.

(Filtration member)

The fiber layers were combined in the same manner as in Example 1 to obtain a filtration member for an air filter.

[Example 7]

(Upstream fiber layer)

30 parts by mass of polyester short fibers of 16 dt in fineness (polyethylene terephthalate, melting point: 265°C), 20 parts by mass of thermally fused polyester short fibers of 8 dt in fineness (core part: polyethylene terephthalate, melting point: 265°C, sheath part: modified polyester, melting point: 155°C), and 50 parts by mass of thermally fused polyester short fibers of 4.4 dt in fineness (core part: polyethylene terephthalate, melting point: 265°C, sheath part: modified polyester, melting point: 180°C) were mixed, and passed through a cotton opening machine and a carding machine to spin a web, and the web was superposed by cross wrap to reach a target mass, and passed through an air-through heating furnace with a conveyor to obtain an upstream layer fiber layer of a thermally bonded nonwoven fabric of 70 g/m$^2$ in basis weight and 0.4 mm in thickness.

(Downstream fiber layer)

A spun-bonded nonwoven fabric was obtained in the same manner as in Example 1, and a processing liquid at pH 7.0 obtained by mixing silver-copper-zinc-supported zeolite ("Agion" AM Slurry manufactured by Sciessent LLC) as an antiviral agent and a nonionic surfactant ("TEXPORT SN-10" manufactured by NICCA CHEMICAL CO., LTD.) as a dispersant in water was applied by spraying, and dried at 110°C to obtain a downstream fiber layer. The concentration of the processing liquid was adjusted such that the adhesion amount of the silver-copper-zinc-supported zeolite was 0.3 g/m$^2$ and such that the adhesion amount of the nonionic surfactant was 0.3 g/m$^2$.
Accordingly, the solid fraction mass ratio between the antiviral agent and the dispersant (solid fraction mass of antiviral agent/the solid fraction mass of dispersant) was 0.3/0.3 = 1.0, and the basis weight of the solid fraction mass of the antiviral agent was 0.3/30 = 0.01 (1.0%) with respect to the basis weight of the downstream fiber layer.

(Filtration member)

The fiber layers were combined in the same manner as in Example 1 to obtain a filtration member for an air filter.

[Example 8]

(Upstream fiber layer)

An upstream fiber layer was obtained in the same manner as in Example 7.

(Downstream fiber layer)

A meltblown nonwoven fabric was obtained in the same manner as in Example 2, and a processing liquid at pH 7.0 obtained by mixing silver-copper-zinc-supported zeolite ("Agion" AM Slurry manufactured by Sciessent LLC) as an antiviral agent and a nonionic surfactant ("TEXPORT SN-10" manufactured by NICCA CHEMICAL CO., LTD.) as a dispersant in water was applied by spraying, and dried at 110°C to obtain a downstream fiber layer. The concentration of the processing liquid was adjusted such that the adhesion amount of the silver-copper-zinc-supported zeolite was 0.3 g/m$^2$ and such that the adhesion amount of the nonionic surfactant was 0.3 g/m$^2$.

Accordingly, the solid fraction mass ratio between the antiviral agent and the dispersant (solid fraction mass of antiviral agent/the solid fraction mass of dispersant) was 0.3/0.3 = 1.0, and the basis weight of the solid fraction mass of the antiviral agent was 0.3/30 = 0.01 (1.0%) with respect to the basis weight of the downstream fiber layer.

(Filtration member)

The fiber layers were combined in the same manner as in Example 1 to obtain a filtration member for an air filter according to Example 8.

[Example 9]

(Upstream fiber layer)

An upstream fiber layer was obtained in the same manner as in Example 7.

(Downstream fiber layer)

A meltblown nonwoven fabric was obtained in the same manner as in Example 2, and a processing liquid at pH 7.0 obtained by mixing silver-copper-zinc-supported zeolite ("Agion" AM Slurry manufactured by Sciessent LLC), a nonionic surfactant ("TEXPORT SN-10" manufactured by NICCA CHEMICAL CO., LTD.) as a dispersant, and a thiabendazole ("Synthol M-30" manufactured by SC Environmental Science Co., Ltd.) to serve as an antifungal agent in water was applied by spraying, and dried at 110°C to obtain a downstream fiber layer. The concentration of the processing liquid was adjusted such that the adhesion amount of the silver-copper-zinc-supported zeolite was 0.3 g/m$^2$, such that the adhesion amount of the nonionic surfactant was 0.3 g/m$^2$, and such that the adhesion amount of the thiabendazole was 0.3 g/m$^2$.

Accordingly, the solid fraction mass ratio between the antiviral agent and the dispersant (solid fraction mass of antiviral agent/the solid fraction mass of dispersant) was 0.3/0.3 = 1.0, and the basis weight of the solid fraction mass of the antiviral agent was 0.3/30 = 0.01 (1.0%) with respect to the basis weight of the upstream fiber layer.

(Filtration member)

The fiber layers were combined in the same manner as in Example 1 to obtain a filtration member for an air filter.

[Example 10]

(Upstream fiber layer)

A thermally bonded nonwoven fabric was obtained in the same manner as in Example 7, a processing liquid at pH 7.0 obtained by mixing a thiabendazole ("Synthol M-30" manufactured by SC Environmental Science **Co.,** Ltd.) to serve as an antifungal agent in water was put in an impregnation tank, and the thermally bonded nonwoven fabric was passed through the impregnation tank to be impregnated with the processing liquid, and then passed through an air-through heating furnace to fabricate an upstream fiber layer. The concentration of the processing liquid was adjusted such that the adhesion amount of the thiabendazole was 0.7 g/m$^2$. Accordingly, the basis weight of the solid fraction mass of the antifungal agent was 0.7/70 = 0.01 (1.0%) with respect to the basis weight of the upstream fiber layer.

(Downstream fiber layer)

A downstream fiber layer was obtained in the same manner as in Example 7.

(Filtration member)

The fiber layers were combined in the same manner as in Example 1 to obtain a filtration member for an air filter.

[Example 11]

(Upstream fiber layer)

An upstream fiber layer was obtained in the same manner as in Example 7.

(Downstream fiber layer)

A spun-bonded nonwoven fabric was obtained in the same manner as in Example 1, a processing liquid was prepared in the same manner as in Example 7, then, a small amount of pH adjuster (weak acid aqueous solution) was added to the liquid to adjust the pH to 5.0, and the processing liquid was applied by spraying to the fabric, and dried at 110°C to obtain a downstream fiber layer. The concentration of the processing liquid was adjusted such that the adhesion amount of the silver-copper-zinc-supported zeolite was 0.3 $g/m^2$ and such that the adhesion amount of the nonionic surfactant was 0.3 $g/m^2$.

Accordingly, the solid fraction mass ratio between the antiviral agent and the dispersant (solid fraction mass of antiviral agent/the solid fraction mass of dispersant) was 0.3/0.3 = 1.0, and the basis weight of the solid fraction mass of the antiviral agent was 0.3/30 = 0.01 (1.0%) with respect to the basis weight of the downstream fiber layer.

(Filtration member)

The fiber layers were combined in the same manner as in Example 1 to obtain a filtration member for an air filter.

[Comparative Example 1]

(Upstream fiber layer)

A thermally bonded nonwoven fabric was obtained in the same manner as in Example 7, a processing liquid at pH 7.0 obtained by mixing silver-copper-zinc-supported zeolite ("Agion" AM Slurry manufactured by Sciessent LLC) as an antiviral agent in water was put in an impregnation tank, and the thermally bonded nonwoven fabric was passed through the impregnation tank to be impregnated with the processing liquid, and then passed through an air-through heating furnace to fabricate an upstream fiber layer. The concentration of the processing liquid was adjusted such that the adhesion amount of the silver-copper-zinc-supported zeolite was 0.7 $g/m^2$.

Accordingly, the basis weight of the solid fraction mass of the antiviral agent was 0.7/70 = 0.01 (1.0%) with respect to the basis weight of the upstream fiber layer.

(Downstream fiber layer)

A downstream fiber layer was obtained in the same manner as in Example 2.

(Filtration member)

The fiber layers were combined in the same manner as in Example 1 to obtain a filtration member for an air filter.

[Comparative Example 2]

(Upstream fiber layer)

An upstream fiber layer was obtained in the same manner as in Example 1 except that the concentration of the processing liquid was adjusted such that the adhesion amount of the silver-copper-zinc-supported zeolite was 0.7 $g/m^2$ and such that the nonionic surfactant was 7.0 $g/m^2$.

Accordingly, the solid fraction mass ratio between the antiviral agent and the dispersant (solid fraction mass of antiviral agent/the solid fraction mass of dispersant) was 0.7/7.0 = 0.10, and the basis weight of the solid fraction mass of the antiviral agent was 0.7/70 = 0.01 (1.0%) with respect to the basis weight of the upstream fiber layer.

(Downstream fiber layer)

A downstream fiber layer was obtained in the same manner as in Example 2.

(Filtration member)

The fiber layers were combined in the same manner as in Example 1 to obtain a filtration member for an air filter.

[Comparative Example 3]

(Upstream fiber layer)

An upstream fiber layer was obtained in the same manner as in Example 7.

(Downstream fiber layer)

A meltblown nonwoven fabric was obtained in the same manner as in Example 2, and a processing liquid at pH 7.0 obtained by mixing a natural plant extract ("Allersave T-70" manufactured by SC Environmental Science Co., Ltd.) as an antiviral agent and a nonionic surfactant ("TEXPORT SN-10" manufactured by NICCA CHEMICAL CO., LTD.) as a dispersant in water was applied by spraying, and dried at 110°C to obtain a downstream fiber layer. The concentration of the processing liquid was adjusted such that the adhesion amount of the natural plant extract was 0.3 g/m$^2$ and such that the adhesion amount of the nonionic surfactant was 0.3 g/m$^2$. It is to be noted that the hydrophilicity of this downstream fiber layer was evaluated as ∘ (water droplets on the fiber layer, with the contact angle of 60° or less).

Accordingly, the solid fraction mass ratio between the antiviral agent and the dispersant (solid fraction mass of antiviral agent/the solid fraction mass of dispersant) was 0.3/0.3 = 1.0, and the basis weight of the solid fraction mass of the antiviral agent was 0.3/30 = 0.01 (1.0%) with respect to the basis weight of the upstream fiber layer.

[Table 1-1]

|  | Upstream layer fiber | Downstream layer fiber | Attachment layer of antiviral agent and dispersant | Antiviral agent | Ratio of antiviral agent basis weight/attachmentside fiber basis weight (%) |
|---|---|---|---|---|---|
| Example 1 | Thermally bonded nonwoven fabric | Spun-bonded non-woven fabric | Upstream fiber layer | Silver-copper-zinc-supported zeolite (metal-based) | 5.0 |
| Example 2 | Thermally bonded nonwoven fabric | Meltblown nonwoven fabric | Upstream fiber layer | Silver-copper-zinc-supported zeolite (metal-based) | 1.0 |
| Example 3 | Thermally bonded nonwoven fabric | Meltblown nonwoven fabric | Upstream fiber layer | Quaternary ammonium salt (ionic organic compound) | 1.0 |
| Example 4 | Thermally bonded nonwoven fabric | Meltblown nonwoven fabric | Upstream fiber layer | Natural plant extract (naturally derived) | 1.0 |
| Example 5 | Thermally bonded nonwoven fabric | Meltblown nonwoven fabric | Upstream fiber layer | Silver-copper-zinc-supported zeolite (metal-based) | 1.0 |
| Example 6 | Thermally bonded nonwoven fabric | Meltblown nonwoven fabric | Upstream fiber layer | Silver-copper-zinc-supported zeolite (metal-based) | 1.0 |

(continued)

| | Upstream layer fiber | Downstream layer fiber | Attachment layer of antiviral agent and dispersant | Antiviral agent | Ratio of antiviral agent basis weight/attachmentside fiber basis weight (%) |
|---|---|---|---|---|---|
| Example 7 | Thermally bonded nonwoven fabric | Spun-bonded non-woven fabric | Downstream fi-ber layer | Silver-copper-zinc-supported zeolite (metal-based) | 1.0 |
| Example 8 | Thermally bonded nonwoven fabric | Meltblown nonwoven fabric | Downstream fiber layer | Silver-copper-zinc-supported zeolite (metal-based) | 1.0 |
| Example 9 | Thermally bonded nonwoven fabric | Meltblown nonwoven fabric | Downstream fiber layer | Silver-copper-zinc-supported zeolite (metal-based) | 1.0 |
| Example 10 | Thermally bonded nonwoven fabric | Spun-bonded non-woven fabric | **Downstream** fi-ber layer | Silver-copper-zinc-supported zeolite (metal-based) | 1.0 |
| Example 11 | Thermally bonded nonwoven fabric | Spun-bonded non-woven fabric | Downstream fi-ber layer | Silver-copper-zinc-supported zeolite (metal-based) | 1.0 |
| Comparative Example 1 | Thermally bonded nonwoven fabric | Meltblown nonwoven fabric | Upstream fiber layer | Silver-copper-zinc-supported zeolite (metal-based) | 1.0 |
| Comparative Example 2 | Thermally bonded nonwoven fabric | Meltblown nonwoven fabric | Upstream fiber layer | Silver-copper-zinc-supported zeolite (metal-based) | 1.0 |
| Comparative Example 3 | Thermally bonded nonwoven fabric | Meltblown nonwoven fabric | Downstream fi-ber layer | Natural plant ex-tract (naturally derived) | 1.0 |

[Table 1-2]

| | Dispersant | Ratio of antiviral agent basis weight/dispersant basis weight (%) | Functional agent attachment layer | Functional agent | Processing liquid pH |
|---|---|---|---|---|---|
| Example 1 | Nonionic surfactant | 1.0 | - | - | 7.0 |
| Example 2 | Nonionic surfactant | 1.0 | - | - | 7.0 |
| Example 3 | Nonionic surfactant | 1.0 | - | - | 7.0 |
| Example 4 | Nonionic surfactant | 1.0 | - | - | 7.0 |

(continued)

| | Dispersant | Ratio of antiviral agent basis weight/dispersant basis weight (%) | Functional agent attachment layer | Functional agent | Processing liquid pH |
|---|---|---|---|---|---|
| Example 5 | Nonionic surfactant | 1.0 | Upstream fiber layer | Thiabendazole (antifungal agent) | 7.0 |
| Example 6 | Nonionic surfactant | 1.0 | Downstream fiber layer | Thiabendazole (antifungal agent) | 7.0 |
| Example 7 | Nonionic surfactant | 1.0 | - | - | 7.0 |
| Example 8 | Nonionic surfactant | 1.0 | - | - | 7.0 |
| Example 9 | Nonionic surfactant | 1.0 | Downstream fiber layer | Thiabendazole (antifungal agent) | 7.0 |
| Example 10 | Nonionic surfactant | 1.0 | Upstream fiber layer | Thiabendazole (antifungal agent) | 7.0 |
| Example 11 | Nonionic surfactant | 1.0 | - | - | 5.0 |
| Comparative Example 1 | - | - | - | - | 7.0 |
| Comparative Example 2 | Nonionic surfactant | 0.10 | - | - | 7.0 |
| Comparative Example 3 | Nonionic surfactant | 1.0 | - | - | 7.0 |

[Table 2]

| | Hydrophilicity evaluation | | Antiviral property | Antibacterial property | Antifungal property | Anti-allergen property |
|---|---|---|---|---|---|---|
| | Upstream fiber layer | Downstream fiber layer | | | | |
| Example 1 | ○ | × | ○ | ○ | × | × |
| Example 2 | ○ | × | ⊙ | ⊙ | △ | × |
| Example 3 | ○ | × | ○ | △ | ○ | × |
| Example 4 | ⊙ | × | ○ | × | × | ⊙ |
| Example 5 | ○ | × | ○ | ○ | ⊙ | × |
| Example 6 | ○ | × | ⊙ | ⊙ | ⊙ | △ |
| Example 7 | ○ | × | ○ | ○ | × | × |
| Example 8 | ○ | × | ⊙ | ⊙ | × | △ |
| Example 9 | ○ | × | ○ | ○ | ⊙ | × |
| Example 10 | ○ | × | ⊙ | ⊙ | ⊙ | △ |
| Example 11 | ○ | × | ⊙ | ⊙ | × | × |
| Comparative Example 1 | △ | × | × | × | × | × |
| Comparative Example 2 | ⊙ | × | △ | △ | × | × |

(continued)

| | Hydrophilicity evaluation | | Antiviral property | Antibacterial property | Antifungal property | Anti-allergen property |
|---|---|---|---|---|---|---|
| | Upstream fiber layer | Downstream fiber layer | | | | |
| Comparative Example 3 | ○ | ○ | △ | × | × | ○ |

INDUSTRIAL APPLICABILITY

The filtration member according to the present invention can be suitably used for air filter applications, particularly as a filters for air purifiers and cabin filters for automobiles.

**Claims**

1.  A filtration member for an antiviral air filter, comprising an upstream fiber layer for which a contact angle with respect to water is 60° or less, and a downstream fiber layer for which a contact angle with respect to water is 90° or greater, wherein the filtration member has an antiviral agent and a dispersant either on the upstream fiber layer or on an upstream-fiber-layer-side surface of the downstream fiber layer, and a solid fraction mass ratio of the antiviral agent to the dispersant ((solid fraction mass of antiviral agent)/(solid fraction mass of dispersant)) is 0.20 to 5.0.

2.  The filtration member for an antiviral air filter according to claim 1, wherein a basis weight of the solid fraction mass of the antiviral agent is 0.01 to 4.0% with respect to a basis weight of the upstream fiber layer or the downstream fiber layer including the antiviral agent.

3.  The filtration member for an antiviral air filter according to claim 1 or 2, wherein the downstream fiber layer is an electret nonwoven fabric.

4.  The filtration member for an antiviral air filter according to any one of claims 1 to 3, wherein the antiviral agent includes a compound including a metal atom.

5.  The filtration member for an antiviral air filter according to any one of claims 1 to 3, wherein the antiviral agent includes an organic compound with ionicity.

6.  The filtration member for an antiviral air filter according to any one of claims 1 to 3, wherein the antiviral agent includes a naturally derived component or a derivative thereof.

7.  The filtration member for an antiviral air filter according to any one of claims 1 to 6, wherein the antiviral agent further has an antibacterial property, an antifungal property, or an anti-allergen property.

8.  The filtration member for an antiviral air filter according to any one of claims 1 to 7, wherein the dispersant is a nonionic surfactant.

9.  The filtration member for an antiviral air filter according to claims 1 to 8, further comprising, besides the antiviral agent, a functional agent that has an antibacterial property, an antifungal property, or an anti-allergen property on the upstream fiber layer or on the upstream-fiber-layer-side surface of the downstream fiber layer.

10.  The filtration member for an air filter according to any one of claims 1 to 9, comprising an intermediate layer including an adsorbent between the upstream fiber layer and the downstream fiber layer.

11.  A method for producing the filtration member for an antiviral air filter according to any one of claims 1 to 10, wherein a processing liquid including an antiviral agent and a dispersant is applied to the upstream fiber layer or an upstream-fiber-layer-side surface of the downstream fiber layer by a processing method of impregnation or spraying.

12.  The method for producing a filtration member for an antiviral air filter according to claim 11, wherein the processing liquid has pH of 3.0 or more and 6.0 or less.

<table>
<tr><td colspan="2">INTERNATIONAL SEARCH REPORT</td><td colspan="2">International application No.<br>**PCT/JP2023/004528**</td></tr>
</table>

**A. CLASSIFICATION OF SUBJECT MATTER**

*B01D 39/16*(2006.01)i; *A01N 25/34*(2006.01)i; *A01N 33/12*(2006.01)i; *A01N 43/78*(2006.01)i; *A01N 59/16*(2006.01)i; *A01N 59/20*(2006.01)i; *A01N 65/00*(2009.01)i; *A01P 1/00*(2006.01)i; *A61L 9/014*(2006.01)i; *A61L 9/16*(2006.01)i; *B03C 3/28*(2006.01)i; *D06M 11/83*(2006.01)i; *D06M 13/152*(2006.01)i; *D06M 13/352*(2006.01)i

FI: B01D39/16 A; A01N25/34 B; A01N33/12; A01N43/78 A; A01N59/16 A; A01N59/16 Z; A01N59/20 Z; A01N65/00 F; A01P1/00; A61L9/014; A61L9/16 F; B03C3/28; D06M11/83; D06M13/152; D06M13/352

According to International Patent Classification (IPC) or to both national classification and IPC

**B. FIELDS SEARCHED**

Minimum documentation searched (classification system followed by classification symbols)

B01D39/16; A01N25/34; A01N33/12; A01N43/78; A01N59/16; A01N59/20; A01N65/00; A01P1/00; A61L9/014; A61L9/16; B03C3/28; D06M11/83; D06M13/152; D06M13/352

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 10-174823 A (MITSUI CHEM., INC.) 30 June 1998 (1998-06-30)<br>entire text, all drawings | 1-12 |
| A | WO 2021/044665 A1 (AIDA CHEMICAL INDUSTRIES CO., LTD.) 11 March 2021 (2021-03-11)<br>entire text, all drawings | 1-12 |
| A | JP 2020-6314 A (NIPPON MUKI CO., LTD.) 16 January 2020 (2020-01-16)<br>entire text, all drawings | 1-12 |
| A | JP 2014-50790 A (NIPPON MUKI CO., LTD.) 20 March 2014 (2014-03-20)<br>entire text, all drawings | 1-12 |
| P, A | JP 2022-58104 A (DAINIPPON PRINTING CO., LTD.) 11 April 2022 (2022-04-11)<br>entire text, all drawings | 1-12 |

☐ Further documents are listed in the continuation of Box C.   ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 March 2023** | **20 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

| International application No. |
|---|
| **PCT/JP2023/004528** |

| Patent document cited in search report | | | Publication date (day/month/year) | Patent family member(s) | | | Publication date (day/month/year) |
|---|---|---|---|---|---|---|---|
| JP | 10-174823 | A | 30 June 1998 | (Family: none) | | | |
| WO | 2021/044665 | A1 | 11 March 2021 | JP | 2021-41303 | A | |
| JP | 2020-6314 | A | 16 January 2020 | (Family: none) | | | |
| JP | 2014-50790 | A | 20 March 2014 | (Family: none) | | | |
| JP | 2022-58104 | A | 11 April 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10000315 A

- JP 2004313755 A